# EUROPEAN PATENT SPECIFICATION

(11) **EP 3 132 755 B1**
(45) Date of publication and mention of the grant of the patent: **05.12.2018**
(21) Application number: 15780491.5
(22) Date of filing: 23.03.2015
(51) Int. Cl.: A61B 17/12

(54) **METAL VASCULAR CLAMP CAPABLE OF BEING DIRECTIONALLY DEGRADED AND ABSORBED AND MANUFACTURING METHOD THEREFOR**
METALLGEFÄSSKLAMMER MIT DIREKTIONALEM ABBAU UND ABSORPTION SOWIE HERSTELLUNGSVERFAHREN DAFÜR
CLAMP VASCULAIRE MÉTALLIQUE POUVANT ÊTRE DÉGRADÉ ET ABSORBÉ DE FAÇON DIRECTIONNELLE, ET SON PROCÉDÉ DE FABRICATION

(30) Priority: 17.04.2014 CN 201410156536
(43) Date of publication of application: 22.02.2017
(73) Proprietor: Suzhou Origin Medical Technology Co., Ltd, Changshu, Jiangsu 215513 (CN)
(72) Inventor: LIU, Jingyi, Changshu Jiangsu 215513 (CN); ZHANG, Shaoxiang, Changshu Jiangsu 215513 (CN); ZHANG, Yuanzhuang, Changshu Jiangsu 215513 (CN); JIANG, Man, Changshu Jiangsu 215513 (CN); XU, Haidong, Changshu Jiangsu 215513 (CN); ZHAO, Changli, Changshu Jiangsu 215513 (CN); ZHANG, Xiaonong, Changshu Jiangsu 215513 (CN)
(74) Representative: Sun, Yiming
(86) International application number: PCT/CN2015/074834
(87) International publication number: WO 2015/158196

(56) References cited:
- EP-A1- 0 128 011
- WO-A1-2013/059826
- CN-A- 1 954 781
- CN-A- 1 954 781
- CN-A- 103 892 884
- CN-U- 202 288 387
- CN-Y- 201 185 950
- CN-Y- 201 185 950
- DE-A1-102004 015 224
- US-A- 4 476 865
- US-A- 4 579 118
- US-A- 5 921 991
- US-A1- 2012 083 803

## Description

### Technology Field of the Invention

This invention relates to an implantable medical device product, more specifically, to a metallic ligating clip and its preparation method used for the permanent vascular closure inside human body in the surgery.

### Background Technology of the Invention

Clinically, surgeons ligate to close the bleeding vessels impossible to suture or unnecessary to suture in the vicinity of surgical spot during the surgical resection surgeries of abdominal cavity and chest, in order to avoid bleeding during operative process and postoperative bleeding. The existing closing technology includes two kinds, such as: 1. suture the parts in need of ligation to closure with thread, disadvantaged by long operating time, big operative difficulty, high requirement on doctor's suture technique and strenuous time-consuming, much damage to patients for long-time operation, and nearly impossibility to operate under endoscopy; 2. adopt ligating clip of metallic titanium and close the ligating clip tight. Such ligating clip can meet the requirements of operating under the endoscopy, but after it is implanted into the patients, titanium metal absorbs x-ray or cause influence on ultrasound to affect postoperative CT and B ultrasonic examination, and the titanium clip cannot be degraded after implanted in vivo and exists permanently; it may even be freed to other place to cause some complications such as inflammatory reaction; 3. polymeric ligating clip for closing adopted in some operations, is also the nondegradable product, with the similar shortages of titanium clip. Therefore, it is of important practical significance and economic value to develop the ligating clip product with biodegradable characteristics by adopting biodegradable metallic materials. The following shows the published patents or utility models representative of the prior art.

The paper of Invention Patent Application No. 200910097174.3 describes a V-shaped synthesis-plastic ligating clip and its fabrication method. Said ligating clip is made of plastic, nondegradable and nonabsorbable, and causes foreign-body reaction; it easily results in translocation phenomenon and damage to other tissues; said ligating clip structure and its fabrication method can only apply to ligating clip made of plastic, its structure designed according to the plastic material, cannot meet the material mechanic properties of biodegradable metallic ligating clip.

The paper of Patent Application No. 200820047583.3 describes a ligating clip is characterized by said self-lock structure being yin-yang fitted self-lock structure or concave-convex fitted self-lock structure. From the view of metal processing & manufacturing, this structure has many machining dead-angles; its fabrication by common machining way is very difficult, and it can only be fabricated by the ways of metal injection molding or powder metallurgy for mass production; its structure and the corresponding processing modes cannot satisfy the mass-production demand for ligating clip of biodegradable metal as the raw material.

The paper of Patent Application No. 201020657032.6 describes a ligating clip is characterized in that said ligating clip is a stainless-steel spring structure and its material cannot be absorbed by degradation; In addition, according to the structure characteristics of ligating clip head, it may rebound and become loose to fall off after implanted into the human body; besides, said ligating clip with exposed sharp part, may cause damage to the surrounding tissues. Moreover, for the sake of elasticity modulus, this structure is suitable for stainless steel material; due to the mechanical properties of biodegradable metal, especially for the reason of low elasticity modulus, this structure is unsuitable for the ligating clip of biodegradable metal.

The paper of Patent Application No. 201120412587.9 describes a tissue-closing clip of absorbable magnesium alloy is characterized by a ligating clip made of magnesium alloy; this clip-body is of an opening at one end and the other parts connected as a whole; both sides in the direction of said clip-body opening are symmetric relatively to the centerline, and the inner wall of said clip-body is opened with through slot. The technical scheme provided by this patent also has many shortcomings, such as 1. the technology scheme mentioned in the patent failed to effectively solve the problem of magnesium degradation, but the severe pitting phenomenon during magnesium metal degradation is the recognized technical difficulty in the field; if cannot be well solved this problem will cause the degradation uncontrolled, greatly increasing the use risk of the device; 2. the technology scheme mentioned in the patent describes a ligating clip structure is similar to the traditional titanium clip, but the tensile strength and plastic performance of magnesium metallic material are far lower than the titanium material; this structure if adopted can lead to the sealing performance after closing cannot meet the practical needs; the clip-body shows fracture failure phenomenon during large-angle plastic deformation; the ligating clip with through slot on the inner-wall, can result in serious tip-potential corrosion phenomenon, and further accelerate the degradation rate at this place, so that the clip body fractures to failure in a short time; so the mentioned patent technology scheme cannot fully meet the actual demand of magnesium-metallic ligating clip.

CN 201 185 950 Y discloses an artery clamp which is in a V-shaped or a U-shaped structure formed by an upper arm and a lower arm, wherein the head portion of the lower arm is provided with a guiding surface which can transversely displace under the longitudinal pressure, and self-locking structures for limiting the longitudinal relaxation motion which are mutually engaged are arranged on the head portion of the upper arm and the head portion of the lower arm. The upper arm and the lower arm of the artery clamp start to close when an applied clamp is closed, the head portion of the upper arm slides on the guiding surface of the head portion of the lower arm under the longitudinal pressure, thereby generating transverse displacement. The self-locking structures which are mutually engaged on the head portion of the upper arm and the head portion of the lower arm for limiting the longitudinal relaxation motion can slide into and lock under the combined action of the restoring force formed after the transverse displacements of the upper arm and the lower arm and the restoring force formed after the transverse displacement of the applied clamp, thereby realizing the closures of the upper arm and the lower arm. The artery clamp not only has strong clamping force, but also avoids the shearing action to tissues in the closure process.

### Summary of the Invention

Specific to overcome the above problems and disadvantages of the prior arts, this invention provides a directionally biodegradable metallic ligating clip and its preparation method for changing the potential difference through directionally changing the microstructure difference at different parts of the ligating clip, so as to realize the directional degradation in different degradation order.

To realize the above purpose, this invention adopts the following technical scheme: **This invention provides a directionally biodegradable metallic ligating clip and its preparation method.** Said metallic ligating clip comprises an upper arm, a lower arm and a tail structure for closing blood vessels; wherein before closing, the upper arm, lower arm and the tail structure is adapted to constitute an asymmetric *V*-shaped structure, and the ends of the upper arm and lower arm are provided with mutually fitted self-lock structures. When using the clip, the blood vessel is placed for closing at the gap between the upper arm and lower arm of the *V*-shaped structure, and through squeezing the upper arm and lower arm, make the included angle gradually decrease until the self-lock structures of the upper arm and lower arm are interlocked and closed mutually. The invention is characterized in that the inner surface and outer surface of said upper arm and lower arm have different microstructures and potentials, wherein the inner surface refers to the ligating clip's part near blood vessel, while the outer surface refers to the ligating clip's part far from blood vessel; the outer surface grain size is larger than the inner surface grain size, and the outer surface has a lower potential than the inner surface, so as to realize the directional degradation in the direction from clip outside to inner surface after the closure of said ligating clip.

In this invention, the adopted material for ligating clip is pure magnesium or magnesium alloy, wherein the content of said pure magnesium is above 99.99%. The adopted biodegradable magnesium metal is enabled to have different degradation performance at its different parts by regulating the processing technique; thus, it can meet the directional degradation requirements of ligating clip whose raw material is biodegradable magnesium, so as to enhance the safety and reliability in the process of degradation.

In this invention, the heads of the upper arm and lower arm of the ligating clip are provided with built-in buckles, and the whole ligating clip has no obvious prominence after clip closing, without damage to soft tissue around the implant position; the whole ligating clip shows a crescent-shaped structure after closing of the upper arm and lower arm, with good elastic structure, good stability after clip closing and nearly no spring back failure; as elongation of biodegradable metallic material is generally low, the ligating clip tail is designed as O-type structure, to avoid its closure effect and degradation performance affected by the tail plasticity deformation during operating ligating clip; said O-ring radius is for ligating clip 0.5-2 times of the thickness from the top of the upper arm to the bottom of lower arm of ligating clip, and the O shape too big may cause the blood vessels to fall in it thus to form poor vascular closure and further postoperative bleeding; while the O shape too small may lack elasticity and induce plasticity deformation of the tail in the process of ligating-clip closing, so as to cause its fracture or affect the degradation stability of O-structure area.

Optimally, said upper-arm and lower-arm bodies have an arc convenient for the upper arm and lower arm to provide stable closure effect.

Said ligating clip is 3.0-20mm long, 1-6mm wide and 0.3-2mm thick, and the arc radius of the upper-arm and lower-arm bodies 5-50mm.

Optimally, the gap between upper arm and lower arm is 0.1-0.5mm after the closure of said ligating clip.

This invention provides a preparation method of above said directionally biodegradable absorbable metallic ligating clip, comprising the following concrete operating steps:
Step 1, Extruding magnesium metal into magnesium sheet;
   Optimally, said magnesium-metal extrusion is happening at temperature between 100°and 300°C and extrusion ratio of 10-100, and the extruded plate grain diameter is ≤20µm.
Step 2, Rolling and annealing magnesium metal sheet for multiple times into a raw material of required thickness;
   Optimally, the deformation of said single rolling is 3%-8%, and the cumulative deformation before annealing is 20%-50%; the middle annealing of sheet is at temperature 130°C-350°C, and the annealing time is 30sec-300sec;
   Optimally, said plate after rolled is 0.3-2mm in thickness.
   Optimally, said rolled plate is of mechanical properties and yield strength >120MPa, tensile strength >150MPa and elongation rate > 4%.
   Optimally, the obtained grain size after said rolling is 1-15µm.
Step 3, Processing with CNC milling machine, linear cutting or laser cutting to obtain the required upper arm, lower arm and O-structure tail;
Step 4, Putting the previously-prepared ligating clip, formed by said upper arm, lower arm and O-structure tail into a directional annealing device for instantaneous heating treatment to form different potential difference between the inner surface and outer surface of the ligating clip, so as to realize the directional degradation function;
   said directional annealing device performs instantaneous heating treatment to directionally change the microstructure of local part material of ligating clip, i.e., both inner surface and outer surface of the upper arm and lower arm of ligating clip have different microstructures and the potential difference; the thickness with different microstructures of both inner surface and outer surface of ligating clip accounts for 20%-80% of the thickness between the inner surface vascular contact surface of ligating clip and outermost edge surface side of ligating clip.

Optimally, said different microstructures are:
(1) For the ligating clip of pure magnesium metal, its different material structures refer to: 1) ligating clip's outer surface grain size is larger than the inner surface grain size; 2) it has different dislocation density and stress level thus to form potential difference on ligating clip in its degradation, so as to ensure the potential difference on the outer surface lower than the inner surface; after the closing of biodegradable ligating clip, the directional degradation proceeds from ligating clip outer surface towards the inner surface (i.e., the part near blood vessel);
(2) For the ligating clip of magnesium alloy, the different material microstructures include: 1) different grain size, 2) the outer surface microstructure has a higher proportion of the second phase precipitated-phase than the inner surface, 3) different dislocation density and stress level thereby to produce the potential difference lower on the outer surface than the inner surface.

Furthermore, the inside-outside potential difference of said ligating clip of pure magnesium or magnesium alloy refers to that the outer surface potential difference is 50-300mV lower than the inner surface, forming a corrosion galvanic-couple during degradation thus to preferentially degrade the outer surface and protect the inner surface, so as to form the needed directional degradation action.

Optimally, said directional annealing refers to that: put ligating clip into the directional annealing device, and spurt the inner surface area of ligating clip with inert gas above the ligating clip for the purpose of cooling; meanwhile, advection heat-exchange tube surrounding ligating clip is around the ligating clip outer surface, into which inlet high-temperature heat-exchange medium, and transfer heat to the ligating clip outer surface for annealing; after a certain period of annealing, change the direction of variable valve for heat-exchange medium, and instantaneously inlet low-temperature medium for cooling; thereby, finish the process of directional annealing and quenching.

Optimally, said directional annealing device comprises the annealing bath, the annealing medium piping laid at the periphery of ligating-clip annealing bath, the annealing medium below set temperature flowing in annealing medium piping, the variable valve at inlet of annealing medium piping and enabling heat-exchange media at different temperatures, and the interconnected medium piping.

Optimally, said inert gas is one or random mixture among nitrogen, helium, neon, argon, krypton, xenon, carbon dioxide.

Optimally, said heat-exchange medium is one of the silicone oil, quenching oil, machine oil, high-temperature high-pressure aqueous solution and high-temperature gas.

Optimally, said annealing temperature is 200°C-400°C, and said thermal annealing time is 10-300 seconds.

Step 5, Electropolishing the previously-prepared ligating clip to remove its surface oxide and impurities.

Compared with the prior art, the present invention has the following beneficial effects:
Said ligating clip in this invention is advantaged by its absorbability to human body, no adverse effects such as foreign body reaction during degradation, and excellent biocompatibility; this invention can ensure not only its effective closure performance and also the fast discharge outside the human body. Its appearance can be designed according to the characteristics of biodegradable metals, with rational structure, and it is of good clip-closing and sealing effect to meet actual clinical demand. The technical scheme provided in the present invention can change the potential difference through directionally changing the microstructure difference at the different parts of the ligating clip, so as to realize the directional degradation in different degradation order. This directional degradation way further ensures the reliability for its clinical use.

### Description of Attached Figures

Please read and refer to the following attached Figures and the detailed description for the unrestricted Embodiments, the other features, purpose and advantages of the present invention will become more obvious:
Figure 1 is a schematic diagram of the biodegradable ligating clip structure;
Figure 2 is a schematic diagram of the biodegradable ligating clip structure-size after closure;
Figure 3 is a schematic diagram of the biodegradable ligating clip structure-size after opening;
Figure 4 is a schematic diagram of the annealing area of the biodegradable ligating clip;
Figure 5 is a schematic diagram of directional degradation of the ligating clip;
Figure 6 is a schematic diagram of directional annealing device layout;
Figure 7 is a schematic diagram of major structure of directional annealing device;
Figure 8 is a schematic diagram of clipping and fixing by biodegradable ligating clipto 4mm blood vessel of pig stomach;

Wherein, 1- clipped blood vessels, 2- ligating clip upper-arm, 3- ligating clip lower-arm, 4- tail O-type structure, 5- upper-arm fixation hook, 6- lower-arm fixation hook, 7- lower-arm elastic structure, 8- inside sealing face of ligating clip, 9- assembled upper fixed-structure, 10- assembled lower fixed-structure, 11- total length of ligating clip, 12- total height of ligating clip, 13- gap after ligating clip closure, 14- arm arc radius of ligating clip, 15- arm included-angle of ligating clip, 16- fixed outer surface area of included angle, 17- fixed inside area of included angle, 18- thickness range of the different microstructures, 19- main body of directional annealing device, 20- ligating clip for annealing, 21- inert gas cooling device, 22- inlet pipe A of heat exchange medium, used for flowing high-temperature heat exchange medium, 23- inlet pipe B of heat exchange medium, used for flowing low-temperature heat exchange medium, 24-outlet pipe A' of heat exchange medium, 25- outlet pipe B' of heat exchange medium, 26- variable valve of heat exchange medium, 27- inlet pipe of heat exchange medium, 28- outlet pipe of heat exchange medium, 29- heat-exchange tube, 30- annealing fixing groove of ligating clip.

### Detailed Description of the Preferred Embodiments

The present invention will now be described in detail hereinafter in combination with the concrete Embodiments. The following Embodiments conduce to the further understanding of this invention by the technical personnel in this field, but don't limit this invention in any form. It should be pointed out that the average technical personnel in this field can make further multiple variants and improvements under the premise of never detaching from the inventive concept. These all belong to the protection scope of this invention.

### Embodiment 1

Extrude the magnesium alloy ingots with magnesium content above 98% into magnesium alloy plate of 3mm thickness, with its extrusion ratio of 25 and extrusion temperature of 300°C, to form the plate grain-size diameter ≤20µm; cold-roll the extrusion-formed plate, and the single cold-rolling deformation amount is 3%; when the cumulative deformation amount is 25%, perform annealing at 300°C temperature and for 200 seconds, and then repeat the above said cold-rolling process until the plate thickness = 0.8mm and its grain-size diameter ≤10µm, tensile strength >180MPa and the elongation >5%.

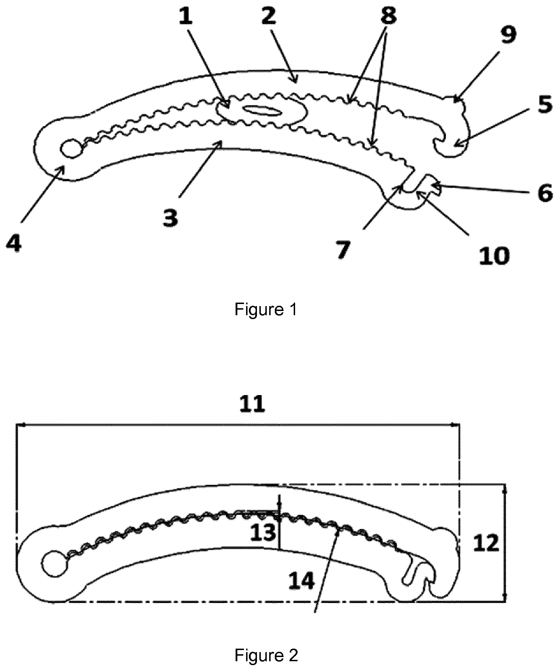

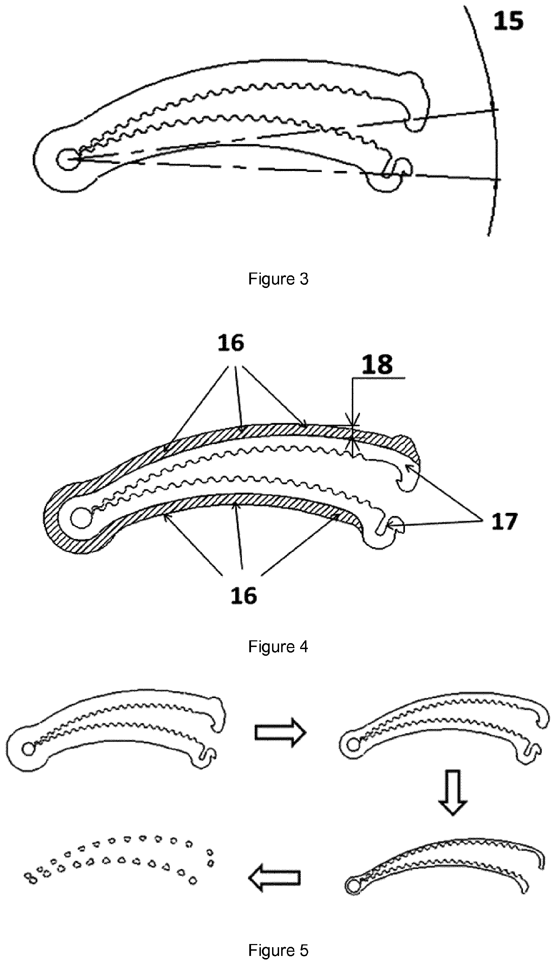

Put the plate obtained from the above processing procedure through the linear cutting machining to get the ligating clip, and the structure is shown in Figure 1 as: 2- ligating clip upper-arm, 3- ligating clip lower-arm, 4- tail O-type structure, 5- upper-arm fixation hook, 6- lower-arm fixation hook, 7- lower-arm elastic structure, 8- inside sealing face of ligating clip, 9-assembled upper fixed-structure, 10-assembled lower fixed-structure. The size is shown in Figure 2 as: total length of 10mm, width of 3mm and thickness of 0.8mm, and the arc diameter of the upper arm and lower arm after closure is 30mm shown as the 14 in Figure 2; the included angle of the upper arm and lower arm is 30°, as shown in Figure 3; the gap of the upper arm and lower arm after closure is 0.2mm, as shown in Figure 2.

In this Embodiment, said upper arm 2 comprises the upper-arm body, 5- upper-arm fixation hook and 9- assembled upper fixed-structure, the 5- and 9- being set at one end away from the 4- tail O-type structure, i.e. at the head; said lower arm 3 comprises the lower-arm body, 6- lower-arm fixation hook and 10- assembled lower fixed-structure, the 6- and 10- being set at one end away from the 4- tail O-type structure, i.e. at the head; the 5- upper-arm fixation hook is a fixation hook bent at the closure side of the 2- upper arm and 3- lower arm, and the 6- lower-arm fixation hook is a fixation hook protruding toward the head of lower-arm body; during the closing of 2-upper arm and 3- lower arm, the 6- lower-arm fixation hook buckles inside of 5- upper-arm fixation hook thus to form the locking.

In this Embodiment, said 5- upper-arm fixation hook and 6- lower-arm fixation hook are arc-shaped on the outer contour; the 5- upper-arm fixation hook inside has an arc space for containing the 6- lower-arm fixation hook, and the shape of this arc space is same to the 6- lower-arm fixation hook; in the process of closing, both fixation hooks (5-, 6-) of the upper arm and lower arm slide along the corresponding arc surface until the 6- lower-arm fixation hook slides into the arc space inside the 5- upper-arm fixation hook, thereby to complete the closing and locking of the 2- upper arm and 3- lower arm.

In this Embodiment, the internal closure side of said upper-arm fixation hook and lower-arm fixation hook, i.e., 8- inside sealing face of ligating clip is provided with wave structure to facilitate better locking and closing, so as to enhance the closure effect.

In this Embodiment, both said upper-arm fixation hook and lower-arm fixation hook with an arc have good overall elastic structure, favorable for the locking and closing of the ligating clip, and facilitating the 2- upper arm and 3- lower arm to present stable closure effect.

In this Embodiment, said lower arm-body is provided with 7- lower-arm elastic structure near 6- lower-arm fixation hook; the 7- lower-arm elastic structure is a groove, this groove being 0.5-4.5mm long and 0.2-2mm wide; 7- lower-arm elastic structure ensure not only enough elastic deformation during the closing of the ligating clip while also adequate locking strength.

In this Embodiment, said 9- assembled upper fixed-structure is a bulge near 5- upper-arm fixation hook, and this bulge arc structure to ensure the 2- upper arm is in fixed connection with the matched device.

In this Embodiment, said 10- assembled lower fixed-structure is set on one side of 7-lower-arm elastic structure, and 6- lower-arm fixation hook is assembled at one end of 10- assembled lower fixed-structure; the other end of 10- assembled lower fixed-structure is an arc-shaped bulge to ensure 3- upper arm is in fixed connection with the matched device.

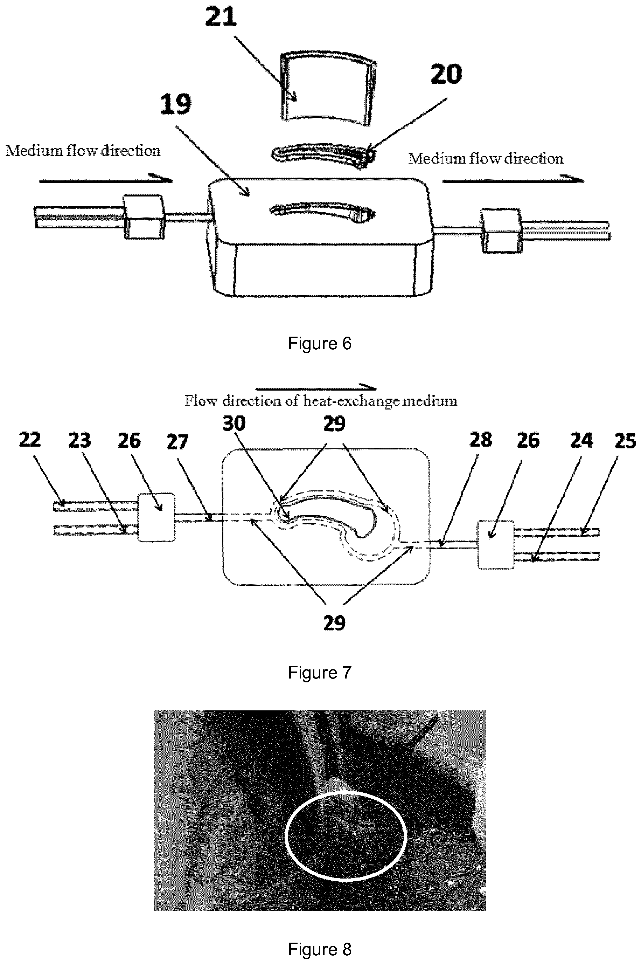

Put the ligating clip for annealing as shown in Figure 6 into the annealing fixing groove of ligating clip of directional annealing-device main-body, shown as the 19 in Figure 6; for the inner surface of the annealing fixing groove of ligating clip shown as the 30 in Figure 7, inlet argon through the inert gas cooling device shown as the 21 in Figure 6, to spurt the ligating-clip inner surface area (17) in Figure 4, in order to keep it cooled and provide the gas protection during annealing; meanwhile, orderly inlet 200°C silicone oil as medium annealing into the heat-exchange medium inlet-pipe A shown as the 22 in Figure 7, variable valve of heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 24 in Figure 7; inlet the high-temperature annealing medium continuously for 60 seconds, and heat and anneal it. Then through switching the variable valve, orderly inlet the normal 8°C silicone oil into the heat-exchange medium inlet-pipe A shown as the 23 in Figure 7, heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 25 in Figure 7; continuously inlet the high-temperature annealing medium for 100 seconds, and quickly cool it to finish the annealing procedure. The grain size in the fixed outer surface area of the ligating clip after annealing shown as the 16 in Figure 4 is 18µm, more than the 10µm in the fixed inner surface area of the ligating clip; partial thickness in the larger grain-size area is 45% of the total area thickness, and for the measured potential difference, the ligating clip outer surface is 100mV lower than the inner surface.

Finally, electropolish the previously-prepared ligating clip for removing the surface impurities to obtain the finished ligating clip, and then soak it into the simulated body fluid at constant temperature 37°C; thereafter, the observation made respectively after 1 week, 2 weeks, 4 weeks and 8 weeks found its real degradation process as shown in Figure 5, showing an obvious outer surface-inner surface directional degradation process.

### Embodiment 2

Extrude the magnesium ingots with magnesium content above 99.99% into magnesium sheet of 3mm thickness, with its extrusion ratio of 25 and extrusion temperature of 300°C, to form the plate grain-size diameter ≤20µm; cold-roll the extrusion-formed plate, and the single cold-rolling deformation amount is 3%; when the cumulative deformation amount is 25%, perform annealing at 300°C temperature and for 200 seconds, and then repeat the above said cold-rolling process until the plate thickness = 0.8mm and its grain-size diameter ≤10µm, tensile strength >180MPa and the elongation >5%.

Put the sheet obtained from the above processing procedure through the linear cutting machining to get the ligating clip, and the structure is shown in Figure 1 as: 2- ligating clip upper-arm, 3- ligating clip lower-arm, 4- tail O-type structure, 5- upper-arm fixation hook, 6- lower-arm fixation hook, 7- lower-arm elastic structure, 8- inside sealing face of ligating clip, 9-assembled upper fixed-structure, 10-assembled lower fixed-structure. The size is shown in Figure 2 as: total length of 10mm, width of 3mm and thickness of 0.8mm, and the arc diameter of the upper arm and lower arm after closure is 30mm as shown at the 14 in Figure 2; the included angle of the upper arm and lower arm is 30°, as shown in Figure 3; the gap of the upper arm and lower arm after closure is 0.2mm, as shown in Figure 2.

Put the ligating clip for annealing as shown in Figure 6 into the annealing fixing groove of ligating clip of directional annealing-device main-body, shown as the 19 in Figure 6; for the inside of the annealing fixing groove of ligating clip shown as the 30 in Figure 7, inlet nitrogen through the inert gas cooling device shown as the 21 in Figure 6, to spurt the ligating-clip inner surface area (17) in Figure 4, in order to keep it cooled and provide the gas protection during annealing; meanwhile, orderly inlet 230°C silicone oil as medium annealing into the heat-exchange medium inlet-pipe A shown as the 22 in Figure 7, variable valve of heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 24 in Figure 7; inlet the high-temperature annealing medium continuously for 60 seconds, and heat and anneal it. Then through switching the variable valve, orderly inlet the normal 15°C silicone oil into the heat-exchange medium inlet-pipe A shown as the 23 in Figure 7, heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 25 in Figure 7; continuously inlet the low-temperature annealing medium for 120 seconds, and quickly cool it to finish the annealing procedure. The grain size in the fixed outer surface area of the ligating clip after annealing shown as the 16 in Figure 4 is 22µm, more than the 12µm in the fixed inner surface area of the ligating clip; partial thickness in the larger grain-size area is 55% of the total area thickness, and for the measured potential difference, the ligating clip outer surface is 150mV lower than the inner surface.

Finally electropolish the previously-prepared ligating clip for removing the surface impurities to obtain the finished ligating clip as shown in Figure 1, and close a silicone tube of 5mm diameter and 0.5 thickness with this finished ligating clip; then soak it into the simulated body fluid at constant temperature 37°C; 2 weeks later, the ligating clip inner surface shown as the 17 in Figure 4, had little change in color and slightly greying, and the width of its upper arm and lower arm reduced by 20%; 5 weeks later, the whole ligating clip was blacking while the width of its upper arm and lower arm reduced by 55% obviously, but the inner surface contour of the ligating clip still remained effective clipping state; 10 weeks after, the ligating clip showed a rupture phenomenon, and the width of its upper arm and lower arm reduced to 20% at rupturing.

### Embodiment 3

Extrude the magnesium ingots with magnesium content above 99.99% into magnesium sheet of 3mm thickness, with its extrusion ratio of 35 and extrusion temperature of 200°C, to form the plate grain-size diameter ≤12µm; cold-roll the extrusion-formed plate, and the single cold-rolling deformation amount is 5%; when the cumulative deformation amount is 40%, perform annealing at 250°C temperature and for 60 seconds, and then repeat the above said cold-rolling process until the plate thickness = 0.8mm and its grain-size diameter ≤4µm, tensile strength >210MPa and the elongation >7%.

Put the plate obtained from the above processing procedure through the linear cutting machining to get the ligating clip, and the structure is shown in Figure 1 as: 2- ligating clip upper-arm, 3- ligating clip lower-arm, 4- tail O-type structure, 5- upper-arm fixation hook, 6- lower-arm fixation hook, 7- lower-arm elastic structure, 8- inside sealing face of ligating clip, 9-assembled upper fixed-structure, 10-assembled lower fixed-structure. The size is shown in Figure 2 as: total length of 10mm, width of 3mm and thickness of 0.8mm, and the arc diameter of the upper arm and lower arm after closure is 30mm as shown at the 14 in Figure 2; the included angle of the upper arm and lower arm is 30°, as shown in Figure 3; the gap of the upper arm and lower arm after closure is 0.2mm, as shown in Figure 2.

Put the ligating clip for annealing as shown in Figure 6 into the annealing fixing groove of ligating clip of directional annealing-device main-body, shown as the 19 in Figure 6; for the inside of the annealing fixing groove of ligating clip shown as the 30 in Figure 7, inlet helium through the inert gas cooling device shown as the 21 in Figure 6, to spurt the ligating-clip inner surface area (17) in Figure 4, in order to keep it cooled and provide the gas protection during annealing; meanwhile, orderly inlet 250°C silicone oil as medium annealing into the heat-exchange medium inlet-pipe A shown as the 22 in Figure 7, variable valve of heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 24 in Figure 7; inlet the high-temperature annealing medium continuously for 80 seconds, and heat and anneal it. Then through switching the variable valve, orderly inlet the normal 8°C silicone oil into the heat-exchange medium inlet-pipe A shown as the 23 in Figure 7, heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 25 in Figure 7; continuously inlet the low-temperature annealing medium for 120 seconds, and quickly cool it to finish the annealing procedure. The grain size in the fixed outer surface area of the ligating clip after annealing shown as the 16 in Figure 4 is 20µm, more than the 12µm in the fixed inner surface area of the ligating clip; partial thickness in the larger grain-size area is 50% of the total area thickness, and for the measured potential difference, the ligating clip outer surface is 120mV lower than the inner surface.

Put the ligating clip for annealing previously-prepared from above procedures into the annealing fixing groove of ligating clip of directional annealing-device main-body, shown as the 30 in Figure 7; shown as the 21 in Figure 6, inlet argon through the inert gas cooling device to spurt the ligating-clip inner surface area, in order to keep it cooled and provide the gas protection during annealing; meanwhile, inlet 250°C silicone oil as medium annealing into the heat-exchanger tube and heat it for 100 seconds; then through switching the variable valve, inlet normal-temperature silicone oil into the heat-exchanger tube to quickly cool it and finish the annealing procedure. The thickness of large-size grain in the ligating clip after annealing shown as the 16 in Figure 4 is 45% of the total area thickness, and for the measured potential difference, the ligating clip outer surface is 120mV lower than the inner surface.

Finally electropolish the previously-prepared ligating clip for removing the surface impurities to obtain the finished ligating clip as shown in Figure 1, and close a silicone tube of 5mm diameter and 0.5 thickness with this finished ligating clip; then soak it into the simulated body fluid at constant temperature 37°C; 2 weeks later, the ligating clip outer surface shown as the 16 in Figure 4 had blacking surface and obvious degradation phenomenon, and the ligating clip inner surface shown as the 17 in Figure 4 had little change in color and slightly greying, while the width of its upper arm and lower arm reduced by 20%; 8 weeks later, the whole ligating clip was blacking and the width of its upper arm and lower arm reduced by 70% obviously, but the inner surface contour of the ligating clip still remained effective clipping state without fracture; 12 weeks after, the ligating clip showed a rupture phenomenon, and the width of its upper arm and lower arm reduced to 15% at rupturing.

### Embodiment 4

Extrude the magnesium ingots with magnesium content above 99.99% into magnesium sheet of 3mm thickness, with its extrusion ratio of 45 and extrusion temperature of 180°C, to form the plate grain-size diameter ≤10µm; cold-roll the extrusion-formed plate, and the single cold-rolling deformation amount is 5.5%; when the cumulative deformation amount is 45%, perform annealing at 200°C temperature and for 40 seconds, and then repeat the above said cold-rolling process until the plate thickness = 0.8mm and its grain-size diameter ≤3µm, tensile strength >230MPa and the elongation >7%.

Put the plate obtained from the above processing procedure through the linear cutting machining to get the ligating clip, and the structure is shown in Figure 1 as: 2- ligating clip upper-arm, 3- ligating clip lower-arm, 4- tail O-type structure, 5- upper-arm fixation hook, 6- lower-arm fixation hook, 7- lower-arm elastic structure, 8- inside sealing face of ligating clip, 9-assembled upper fixed-structure, 10-assembled lower fixed-structure. The size is shown in Figure 2 as: total length of 10mm, width of 3mm and thickness of 0.8mm, and the arc diameter of the upper arm and lower arm after closure is 30mm as shown at the 14 in Figure 2; the included angle of the upper arm and lower arm is 30°, as shown in Figure 3; the gap of the upper arm and lower arm after closure is 0.2mm, as shown in Figure 2.

Put the ligating clip for annealing as shown in Figure 6 into the annealing fixing groove of ligating clip of directional annealing-device main-body shown as the 19 in Figure 6; for the inside of the annealing fixing groove of ligating clip shown as the 30 in Figure 7, inlet carbon dioxide (CO₂) through the inert gas cooling device shown as the 21 in Figure 6, to spurt the ligating-clip inner surface area (17) in Figure 4, in order to keep it cooled and provide the gas protection during annealing; meanwhile, orderly inlet 270°C silicone oil as medium annealing into the heat-exchange medium inlet-pipe A shown as the 22 in Figure 7, variable valve of heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 24 in Figure 7; inlet the high-temperature annealing medium continuously for 12 seconds, and heat and anneal it. Then through switching the variable valve, orderly inlet the normal 10°C silicone oil into the heat-exchange medium inlet-pipe A shown as the 23 in Figure 7, heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 25 in Figure 7; continuously inlet the low-temperature annealing medium for 200 seconds, and quickly cool it to finish the annealing procedure. The grain size in the fixed outer surface area of the ligating clip shown as the 16 in Figure 4 after annealing is 25µm, more than the 13µm in the fixed inner surface area of the ligating clip; partial thickness in the larger grain-size area is 60% of the total area thickness, and for the measured potential difference, the ligating clip outer surface is 150mV lower than the inner surface.

Finally electropolish the previously-prepared ligating clip for removing the surface impurities to obtain the finished ligating clip as shown in Figure 1, and close a silicone tube of 5mm diameter and 0.5 thickness with this finished ligating clip; then soak it into the simulated body fluid at constant temperature 37°C; 2 weeks later, the ligating clip outer surface shown as the 16 in Figure 4 had blacking surface and obvious degradation phenomenon, and the ligating clip inner surface shown as the 17 in Figure 4 had little change in color and slightly greying, while the width of its upper arm and lower arm reduced by 25%; 6 weeks later, the whole ligating clip was blacking and the width of its upper arm and lower arm reduced by 60% obviously, but the inner surface contour of the ligating clip still remained effective clipping state without fracture; 11 weeks after, the ligating clip showed a rupture phenomenon, and the width of its upper arm and lower arm reduced to 15% at rupturing.

### Embodiment 5

Extrude the magnesium ingots with magnesium content above 99.99% into magnesium sheet of 2mm thickness, with its extrusion ratio of 50 and extrusion temperature of 200°C, to form the plate grain-size diameter ≤10µm; cold-roll the extrusion-formed plate, and the single cold-rolling deformation amount is 7%; when the cumulative deformation amount is 55%, perform annealing at 180°C temperature and for 60 seconds, and then repeat the above said cold-rolling process until the plate thickness = 0.6mm and its grain-size diameter ≤3µm, tensile strength >230MPa and the elongation >7%.

Put the plate obtained from the above processing procedure through the linear cutting machining to get the ligating clip, and the structure is shown in Figure 1 as: 2- ligating clip upper-arm, 3- ligating clip lower-arm, 4- tail O-type structure, 5- upper-arm fixation hook, 6- lower-arm fixation hook, 7- lower-arm elastic structure, 8- inside sealing face of ligating clip, 9-assembled upper fixed-structure, 10-assembled lower fixed-structure. The size is shown in Figure 2 as: total length of 6mm, width of 2mm and thickness of 0.6mm, and the arc diameter of the upper arm and lower arm after closure is 20mm as shown at the 14 in Figure 2; the included angle of the upper arm and lower arm is 40° as shown in Figure 3; the gap of the upper arm and lower arm after closure is 0.15mm, as shown in Figure 2.

Put the ligating clip for annealing as shown in Figure 6 into the annealing fixing groove of ligating clip of directional annealing-device main-body shown as the 19 in Figure 6; for the inside of the annealing fixing groove of ligating clip shown as the 30 in Figure 7, inlet neon through the inert gas cooling device shown as the 21 in Figure 6, to spurt the ligating-clip inner surface area (17) in Figure 4, in order to keep it cooled and provide the gas protection during annealing; meanwhile, orderly inlet 180°C silicone oil as medium annealing into the heat-exchange medium inlet-pipe A shown as the 22 in Figure 7, variable valve of heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 24 in Figure 7; inlet the high-temperature annealing medium continuously for 60 seconds, and heat and anneal it. Then through switching the variable valve, orderly inlet the normal 12°C silicone oil into the heat-exchange medium inlet-pipe A shown as the 23 in Figure 7, heat-exchange medium shown as the 26 in Figure 7, heat-exchange medium inlet-pipe shown as the 27 in Figure 7, heat-exchanger tube shown as the 29 in Figure 7, heat-exchange medium outlet-pipe shown as the 28 in Figure 7, variable valve of heat-exchange medium shown as the 26 in Figure 7 and the heat-exchange medium outlet-pipe shown as the 25 in Figure 7; continuously inlet the low-temperature annealing medium for 120 seconds, and quickly cool it to finish the annealing procedure. The grain size in the fixed outer surface area of the ligating clip shown as the 16 in Figure 4 after annealing is 18µm, more than the 12µm in the fixed inner surface area of the ligating clip; partial thickness in the larger grain-size area is 40% of the total area thickness, and for the measured potential difference, the ligating clip outer surface is 90mV lower than the inner surface.

Finally electropolish the previously-prepared ligating clip for removing the surface impurities to obtain the finished ligating clip; then close a blood vessel of 4mm diameter in rabbit stomach with this finished ligating clip; after 8 weeks of its implantation, the findings from the dissection to the experimental object is that the surface of the blood vessel showed blacking and obvious degradation indication, and the width of its upper arm and lower arm reduced by 60%, but the inner surface contour of the ligating clip still remained effective clipping state without fracture indication;

From the above, this present invention can change the potential difference through directionally changing microstructure difference at different parts of ligating clip, by using the biodegradable absorption performance of biodegradable metal, so as to enable the ligating clip to orderly degrade as per the preset sequence; thereby, the directional degradation in different degradation sequence is realized. What should be understood is that although this present invention is only on the magnesium Embodiment, the other biodegradable metals with biocompatibility also applies to this present invention, which is realizable for the technical personnel in this field.

## Claims

1. A directionally biodegradable metallic ligating clip, said ligating clip is made of biodegradable metal, comprising an upper arm (2) for closing blood vessels, a lower arm (3) and a tail structure (4) for closing blood vessels; wherein before their closing, the upper arm, the lower arm and the tail structure are adapted to form a *V-*shape structure, wherein the upper arm and the lower arm are designed with self-lock structures at their ends, wherein when using the clip, a blood vessel is placed between the upper arm and the lower arm of the *V*-shaped structure, and the upper arm and lower arm are pressed to gradually decrease an included angle (15) between the arms until the self-lock structures of the upper arm and the lower arm are interlocked and closed mutually;
**said clip being characterized in that:**
said tail structure is an O-type and **in that** an inner and an outer surface of said upper arm and
lower arm have different microstructures and potentials, wherein the inner surface refers to the part of ligating clip near the blood vessel, and the outer surface refers to the part of the ligating clip farther away from the blood vessel, wherein a grain size in said outer surfaces is larger than a grain size in said inner surfaces, and the outer surface has a lower degradation potential than the inner surface, so as to realize a directional degradation in a direction from the outer surface to the inner surface after the closure of said clip.

2. The directionally biodegradable metallic ligating clip according to claim 1, wherein said self-lock structures of the upper arm and the lower arm are built-in buckles, and the whole ligating clip has no obvious prominence after the clip is closed; the whole ligating clip showing a crescent-shaped resilient structure after closing of the upper arm and lower arm, having an elastic tail O-type structure with an O-ring radius having a thickness of 0.5-2 times the thickness from top of the upper arm to bottom of the lower arm of the clip, when said clip is closed.

3. The directionally biodegradable metallic ligating clip according to claim 1, wherein said upper arm comprises the upper-arm body, the lock structure and an assembled upper fixed structure (9), the upper-arm lock structure and the assembled upper fixed structure set at the head of the upper-arm body; said lower arm comprises the lower-arm body, the lock structure and an assembled lower fixed structure (10), the lower-arm lock structure and the assembled lower fixed structure set at the head of lower-arm body; the upper-arm lock structure fits mutually with the lower-arm lock structure,
wherein said upper-arm lock structure is a fixation hook (5) bending to the closing side of the upper arm and lower arm, and the lower-arm lock structure is a fixation hook (6) protruding to the head of lower-arm body; when closing the upper arm and lower arm, the fixation hook of lower-arm buckles into that of the upper arm thereby to form locking; said fixation hooks of the upper arm and lower arm are arc-shaped on the outer contour; the upper-arm fixation hook inside has an arc space for containing the lower-arm fixation hook, and the shape of this arc space is same to the lower-arm fixation hook; in the process of closing, both fixation hooks of the upper arm and lower arm slide along the corresponding arc surface until the lower-arm fixation hook slides into the arc space inside the upper-arm fixation hook, thereby allowing the completion of the closing and locking of the upper arm and lower arm.

4. A preparation method of directionally biodegradable metallic ligating clip according to claim 1, said method comprising the use of magnesium as the biodegradable metal, with the following concrete steps:
Step 1, Extruding magnesium ingots into magnesium sheet;
Step 2, Rolling and annealing magnesium sheet for multiple times into a raw material of required thickness;
Step 3, Processing with CNC milling machine, linear cutting or laser cutting to obtain the required upper arm, lower arm and O-structure tail;
Step 4, Putting the previously-prepared ligating clip, formed by said upper arm, lower arm and O-structure tail into a directional annealing device for instantaneous heating treatment to form different degradation potential between the inner and outer surface of the clip, so as to realize the directional degradation function;
Said directional annealing device adapted to perform instantaneous heating treatment to directionally change the microstructures of local part material of clip, in that both inner and outer surface of the upper arm and lower arm of ligating clip have different microstructures and such that a grain size in said outer surfaces is larger than a grain size in said inner surfaces, the thickness of said different microstructures of both inner and outer surface of ligating clip accounts for 20%-80% of the thickness between the inside vascular contact surface of said ligating clip and outermost edge surface of said ligating clip;
Step 5, Electropolishing the previously-prepared ligating clip to remove its surface oxide and impurities.

5. The preparation method of directionally biodegradable metallic ligating clip according to claim 4, is **characterized in that:** in step 1, said magnesium-metal extrusion is happening at temperature between 100°and 300°C and extrusion ratio of 10-100, and the extruded sheet grain diameter is ≤20µm.

6. The preparation method of directionally biodegradable metallic ligating clip according to claim 4, is **characterized in that:** in step 2, the deformation of said single rolling is 3%-8%, and the cumulative deformation before annealing is 20%-50%; the middle annealing of sheet is at temperature 130°C-350°C, with annealing time of 30-300sec;
wherein said rolled sheet is of 0.3-2mm thickness, and of the mechanical properties and yield strength >120MPa, tensile strength >150MPa and elongation > 4%; wherein the obtained grain size after said rolling is 1-15µm.

7. The preparation method of directionally biodegradable metallic ligating clip according to claim 4, is **characterized in that:** in step 4, said different microstructures refer to:
(1) For the ligating clip of pure magnesium, the different material microstructures refer to: 1) the outer surface grain size of the ligating clip is larger than the inner surface grain size; 2) it has different dislocation densities and stress level thus to form degradation potential on ligating clip in its degradation, so as to ensure the degradation potential on the outer surface is lower than on the inner surface; after the closing of biodegradable clip, the directional degradation proceeds from the ligating clip outside towards the inside;
(2) For the ligating clip of magnesium alloy, the different material microstructures include: 1) different grain size, 2) the outer surface microstructure has a higher proportion of the second phase precipitated-phase than the inner surface, 3) different dislocation density and stress level thereby to produce the degradation potential difference lower on the outer surface than the inner surface.

8. The preparation method of directionally biodegradable metallic ligating clip according to claim 7, is **characterized in that:** the inside-outside potential difference of said ligating clip of pure magnesium or magnesium alloy refers to that the outside potential difference is 50-300mV lower than the inside, forming a corrosion galvanic-couple during degradation thus to preferentially degrade the outside and protect the inside, so as to form the needed directional degradation action.

9. The preparation method of directionally biodegradable metallic ligating clip according to any one of claims 4 to 7, is **characterized in that:** said directional annealing refers to that: put ligating clip into the directional annealing device, and spurt the inside area of ligating clip with inert gas above the ligating clip for the purpose of cooling; meanwhile, advection heat-exchange tube surrounding ligating clip is around the ligating clip outside, into which inlet 200°-410°C high-temperature heat-exchange medium, and transfer heat to the ligating clip outside for annealing; after a certain period of annealing, change the direction of a variable valve for heat-exchange medium, and instantaneously inlet 5°C-30°C low-temperature medium for cooling; thereby, finish the process of directional annealing and quenching.

10. The preparation method of directionally biodegradable metallic ligating clip according to claim 9, is **characterized in that:** said directional annealing device comprises an annealing bath, an annealing medium piping laid at the periphery of clip annealing bath, the annealing medium at set temperature flowing in annealing medium piping, the variable valve at inlet of annealing medium piping enabling media heat-exchange at different temperatures, and the interconnected medium piping;
Said inert gas is one or random mixture among nitrogen, helium, neon, argon, krypton, xenon, carbon dioxide;
Said heat-exchange medium is one of silicone oil, quenching oil, machine oil, high-temperature high-pressure aqueous solution and high-temperature gas;
Said annealing temperature is 200°C-400°C, and said thermal annealing time is 10-300 seconds.

## Patentansprüche

1. Direktional biologisch abbaubare metallische Ligaturklammer, wobei die Ligaturklammer aus biologisch abbaubarem Metall hergestellt ist, umfassend einen oberen Arm (2) zum Verschließen von Blutgefäßen, einen unteren Arm (3) und eine Endstückstruktur (4) zum Verschließen von Blutgefäßen; wobei vor deren Verschließen der obere Arm, der untere Arm und die Endstückstruktur angepasst sind, eine V-förmige Struktur zu bilden, wobei der obere Arm und der untere Arm an ihren Enden mit selbstsichernden Strukturen ausgebildet sind, wobei bei Verwendung der Klammer ein Blutgefäß zwischen dem oberen Arm und dem unteren Arm der V-förmigen Struktur platziert wird, und der obere Arm und der untere Arm gedrückt werden, um nach und nach einen zwischen den Armen eingeschlossenen Winkel (15) zu verkleinern, bis die selbstsichernden Strukturen des obere Arms und untere Arms miteinander verbunden und geschlossen werden;
wobei die Klammer **dadurch gekennzeichnet ist, dass**:
die Endstückstruktur von einem O-Typ ist, und dass eine innere und eine äußere Fläche des oberen Arms und unteren Arms unterschiedliche Mikrostrukturen und Potentiale aufweisen, wobei sich die innere Fläche auf den Teil der Ligaturklammer nahe dem Blutgefäß bezieht und sich die äußere Fläche auf den Teil der Ligaturklammer weiter weg von dem Blutgefäß bezieht, wobei eine Korngröße in den äußeren Flächen größer als eine Korngröße in den inneren Flächen ist, und die äußere Fläche ein niedrigeres Abbaupotential aufweist als die innere Fläche, um nach dem Schließen der Klammer eine direktionale Degradation in einer Richtung von der äußeren Fläche zu der inneren Fläche zu realisieren.

2. Direktional biologisch abbaubare metallische Ligaturklammer nach Anspruch 1, wobei die selbstsichernden Strukturen des oberen Arms und des unteren Arms integrierte Ausbeulungen sind und die gesamte Ligaturklammer keine deutliche Prominenz aufweist, nachdem die Klammer geschlossen ist; wobei die gesamte Ligaturklammer nach dem Verschließen des oberen Arms und des unteren Arms eine halbmondförmige elastische Struktur zeigt, mit einer elastischen Endstückstruktur vom O-Typ mit einem O-Ring-Radius mit einer Dicke des 0,5 bis 2-fachen der Dicke von der Oberseite des oberen Arms bis zur Unterseite des unteren Arms der Klammer, wenn die Klammer geschlossen ist.

3. Direktional biologisch abbaubare metallische Ligaturklammer nach Anspruch 1, wobei der obere Arm den oberen Armkörper, die Verriegelungsstruktur, und eine eingebaute obere feste Struktur (9) umfasst, wobei die Verriegelungsstruktur des oberen Arms und die eingebaute obere feste Struktur am Kopf des oberen Armkörpers angesetzt sind; der untere Arm den unteren Armkörper, die Verriegelungsstruktur, und eine eingebaute untere feste Struktur (10) umfasst, wobei die Verriegelungsstruktur des unteren Arms und die eingebaute untere feste Struktur am Kopf des unteren Armkörpers angesetzt sind; die Verriegelungsstruktur des oberen Arms mit der Verriegelungsstruktur des unteren Arms gegenseitig zusammenpasst,
wobei die Verriegelungsstruktur des oberen Arms ein Fixierhaken (5) ist, der sich zu der Schließseite des oberen Arms und des unteren Arms biegt, und die Verriegelungsstruktur des unteren Arms ein Fixierhaken (6) ist, der an dem Kopf des unteren Armkörpers vorsteht; wenn der obere Arm und der untere Arm geschlossen werden, der Fixierhaken des unteren Arms in den des oberen Arms einschnappt, um dadurch eine Verriegelung zu bilden;
die Fixierhaken des oberen Arms und des unteren Arms an der Außenkontur bogenförmig sind; der Fixierhaken des oberen Arms einen Bogenraum aufweist, um den Fixierhaken des unteren Arms aufzunehmen, und die Form dieses Bogenraums dem Fixierhaken des Unterarms gleicht; während des Vorgangs des Schließens beide Fixierhaken des oberen Arms und des unteren Arms entlang der entsprechenden Bogenfläche gleiten, bis der Fixierhaken des unteren Arms in den Bogenraum innerhalb des Fixierhakens des oberen Arms gleitet, wodurch das Verschließen und die Verriegelung des oberen Arms und des unteren Arms ermöglicht wird.

4. Herstellungsverfahren einer direktional biologisch abbaubaren metallischen Ligaturklammer nach Anspruch 1, wobei das Verfahren die Verwendung von Magnesium als biologisch abbaubares Metall umfasst, mit den folgenden konkreten Schritten:
Schritt 1, Extrudieren von Magnesiumbarren in Magnesiumfolie;
Schritt 2, mehrmaliges Walzen und Tempern von Magnesiumblech, zu einem Rohmaterial mit der erforderlichen Dicke;
Schritt 3, Bearbeiten mit einer CNC-Fräsmaschine, lineares Schneiden oder Laserschneiden, um den erforderlichen oberen Arm, unteren Arm, und das O-Struktur-Endstück zu erhalten;
Schritt 4, Einsetzen der zuvor hergestellten Ligaturklammer, die durch den oberen Arm, den unteren Arm, und das O-Struktur-Endstück gebildet wird, in eine direktionale Tempervorrichtung zur sofortigen Wärmebehandlung, um ein zwischen der inneren und äußeren Fläche der Klammer unterschiedliches Degradationspotential zu bilden, um die direktionale Degradationsfunktion zu realisieren;
wobei die direktionale Tempervorrichtung angepasst ist, eine sofortige Wärmebehandlung durchzuführen, um die Mikrostrukturen des Materials des lokalen Teils der Klammer in einer Richtung zu ändern, indem sowohl die innere als auch die äußere Fläche des oberen Arms und des unteren Arms der Ligaturklammer unterschiedliche Mikrostrukturen aufweisen und so eine Korngröße in den äußeren Flächen größer als eine Korngröße in den inneren Flächen ist, wobei die Dicke der unterschiedlichen Mikrostrukturen sowohl der inneren als auch der äußeren Fläche der Ligaturklammer 20% bis 80% der Dicke zwischen der inneren vaskulären Kontaktfläche der Ligaturklammer und der Fläche am äußersten Rand der Ligaturklammer beträgt;
Schritt 5, Elektropolieren der zuvor hergestellten Ligaturklammer, um deren Flächenoxid und Verunreinigungen zu entfernen.

5. Herstellungsverfahren einer direktional biologisch abbaubaren metallischen Ligaturklammer nach Anspruch 4, **dadurch gekennzeichnet, dass**: in Schritt 1 die Magnesium-Metall-Extrusion bei einer Temperatur zwischen 100° und 300°C und einem Extrusionsverhältnis von 10 bis 100 erfolgt, und der Korndurchmesser des extrudierten Blechs ≤ 20 µm ist.

6. Herstellungsverfahren einer direktional biologisch abbaubaren metallischen Ligaturklammer nach Anspruch 4, **dadurch gekennzeichnet, dass**: in Schritt 2 die Verformung eines einzigen Walzens 3% bis 8% beträgt und die kumulative Verformung vor dem Tempern 20% bis 50% beträgt; das mittlere Tempern des Blechs eine Temperatur von 130 °C bis 350 °C aufweist, mit einer Temperzeit von 30 bis 300 Sek.;
wobei das gewalzte Blatt eine Dicke von 0,3 bis 2 mm aufweist, und mechanische Eigenschaften und eine Streckgrenze > 120 MPa, eine Zugfestigkeit > 150 MPa, und eine Dehnung > 4% aufweist; wobei die erhaltene Korngröße nach dem Walzen 1 bis 15 µm beträgt.

7. Herstellungsverfahren einer direktional biologisch abbaubaren metallischen Ligaturklammer nach Anspruch 4, **dadurch gekennzeichnet, dass** sich in Schritt 4 die unterschiedlichen Mikrostrukturen beziehen auf:
(1) für die Ligaturklammer aus reinem Magnesium beziehen sich die unterschiedlichen Materialmikrostrukturen auf: 1) die Korngröße der äußeren Fläche der Ligaturklammer ist größer als die Korngröße der inneren Fläche; 2) sie weist unterschiedliche Versetzungsdichten und Spannungsniveaus auf, um somit bei der Degradation der Ligaturklammer ein Degradationspotential zu bilden, um so zu gewährleisten, dass das Potential auf der äußeren Fläche niedriger ist als auf der inneren Fläche; nach dem Verschließen der biologisch abbaubaren Klammer verläuft der direktionale Abbau der Ligaturklammer von außen nach innen;
(2) für die Ligaturklammer aus Magnesiumlegierung weisen die unterschiedlichen Materialmikrostrukturen auf: 1) eine unterschiedliche Korngröße, 2) die Mikrostruktur der äußere Fläche hat einen höheren Anteil der zweiten Phase, ausgefällten Phase, als die innere Fläche, 3) die Versetzungsdichte und das Spannungsniveau sind unterschiedliche, um dadurch die Degradationspotentialdifferenz auf der äußeren Fläche niedriger als auf der inneren Fläche zu erzeugen.

8. Herstellungsverfahren einer direktional biologisch abbaubaren metallischen Ligaturklammer nach Anspruch 7, **dadurch gekennzeichnet, dass**: die Innen-Außen-Potentialdifferenz der Ligaturklammer aus reinem Magnesium oder einer Magnesiumlegierung sich darauf bezieht, dass die äußere Potentialdifferenz 50 bis 300 mV niedriger ist als die innere ist, wobei ein galvanisches Korrosions-Paar gebildet wird während der Degradation, so dass vorzugsweise die Außenseite abgebaut wird und die Innenseite geschützt wird, um die erforderliche direktionale Degradationswirkung zu bilden.

9. Herstellungsverfahren einer direktional biologisch abbaubaren metallischen Ligaturklammer nach einem der Ansprüche 4 bis 7, **dadurch gekennzeichnet, dass** das direktionale Tempern sich bezieht auf: Einsetzen der Ligaturklammer in die direktionale Tempervorrichtung, und Besprühen des Innenbereichs der Ligaturklammer mit Inertgas über der Ligaturklammer zum Zweck der Kühlung; währenddessen umgibt ein Advektions-Wärmetauschrohr die Ligaturklammer von außen, um die Ligaturklammer herum, in das ein 200 ° bis 410 °C heißes Hochtemperatur-Wärmeaustauschmedium eingelassen wird, und übertragt Wärme zum Tempern an das Äußere der Ligaturklammer; nach einer bestimmten Temperperiode, Wechseln der Richtung eines variablen Ventils für das Wärmeaustauschmedium, und sofortiges Einlassen eines Niedertemperaturmediums mit 5 °C bis 30 °C zum Kühlen; Beenden des Prozesses des direktionalen Temperns und Abschrecken.

10. Herstellungsverfahren nach 9, **dadurch gekennzeichnet, dass**: die direktionale Tempervorrichtung ein Temperbad, eine Tempermediumrohrleitung, das am Rand des Klammertemperbades verlegt ist, das Tempermedium, das mit der eingestellten Temperatur in der Tempermediumrohrleitung strömt, das variablen Ventil am Einlass der Tempermediumrohrleitung, das den Wärmeaustausch der Medien bei unterschiedlichen Temperaturen ermöglicht, und die zwischengeschaltete Mediumrohrleitung umfasst;
das Inertgas eine oder eine zufällige Mischung aus Stickstoff, Helium, Neon, Argon, Krypton, Xenon, Kohlendioxid ist;
das Wärmeaustauschmedium eines von Silikonöl, Abschrecköl, Maschinenöl, einer wässrigen Hochtemperatur-Hochdrucklösung, und einem Hochtemperaturgas ist;
die Tempertemperatur 200 °C bis 400 °C beträgt und die thermische Temperzeit 10 bis 300 Sekunden beträgt.

## Revendications

1. Clip métallique de ligature biodégradable directionnellement, ledit clip de ligature étant composé d'un métal biodégradable, comprenant un bras supérieur (2) pour fermer les vaisseaux sanguins, un bras inférieur (3) et une structure de queue (4) pour fermer les vaisseaux sanguins ; sachant que, avant leur fermeture, le bras supérieur, le bras inférieur et la structure de queue sont aptes à former une structure en forme de V, le bras supérieur et le bras inférieur étant conçus avec des structures autobloquantes à leurs extrémités, sachant que, lors de l'utilisation du clip, un vaisseau sanguin est placé entre le bras supérieur et le bras inférieur sur la structure en forme de V, et que le bras supérieur et le bras inférieur sont comprimés pour diminuer graduellement un angle inclus (15) entre les bras jusqu'à ce que les structures autobloquantes du bras supérieur et du bras inférieur soient imbriquées et fermées mutuellement ;
ledit clip étant **caractérisés en ce que** :
ladite structure de queue est de type torique et **en ce qu'**une surface intérieure et une surface extérieure desdits bras supérieur et bras inférieur ont des microstructures et des potentiels différents, la surface intérieure se référant à la partie du clip de ligature proche du vaisseau sanguin, et la surface extérieure se référant à la partie du clip de ligature plus éloignée du vaisseau sanguin, une granulométrie dans lesdites surfaces extérieures étant supérieure à une granulométrie dans lesdites surfaces intérieures, et la surface extérieure ayant un potentiel de dégradation plus faible que la surface intérieure de manière à réaliser une dégradation directionnelle dans un sens allant de la surface extérieure vers la surface intérieure après la fermeture dudit clip.

2. Clip métallique de ligature biodégradable directionnellement selon la revendication 1, dans lequel lesdites structures autobloquantes du bras supérieur et du bras inférieur sont des boucles intégrées, et l'ensemble du clip de ligature n'est pas apparemment proéminent une fois que le clip est fermé ; l'ensemble du clip de ligature présentant une structure résiliente de forme croissante après la fermeture du bras supérieur et du bras inférieur, ayant une structure de type torique à queue élastique dotée d'un rayon de joint torique ayant une épaisseur représentant 0,5 à 2 fois l'épaisseur depuis le dessus du bras supérieur jusqu'au bas du bras inférieur du clip lorsque ledit clip est fermé.

3. Clip métallique de ligature biodégradable directionnellement selon la revendication 1, dans lequel ledit bras supérieur comprend le corps de bras supérieur, la structure de verrouillage et une structure fixe supérieure assemblée (9), la structure de verrouillage de bras supérieur et la structure fixe supérieure assemblée étant posées à la tête du corps de bras supérieur ; ledit bras inférieur comprenant le corps de bras inférieur, la structure de verrouillage et une structure fixe inférieure assemblée (10), la structure de verrouillage de bras inférieur et la structure fixe inférieure assemblée étant posées à la tête du corps de bras inférieur ; la structure de verrouillage de bras supérieur s'ajustant mutuellement avec la structure de verrouillage de bras inférieur,
ladite structure de verrouillage de bras supérieur étant un crochet de fixation (5) recourbé vers le côté fermeture du bras supérieur et du bras inférieur, et la structure de verrouillage de bras inférieur étant un crochet de fixation (6) dépassant de la tête du corps de bras inférieur ; lors de la fermeture du bras supérieur et du bras inférieur, le crochet de fixation du bras inférieur se bouclant dans celui du bras supérieur pour établir ainsi un verrouillage ;
lesdits crochets de fixation du bras supérieur et du bras inférieur étant de forme arquée sur leur contour extérieur ; la crochet de fixation du bras supérieur comportant un espace arqué pour contenir le crochet de fixation du bras inférieur, et la forme de cet espace arqué étant la même que celle du crochet de fixation inférieur ; lors du processus de fermeture, les deux crochets de fixation du bras supérieur et du bras inférieur glissant le long de la surface arquée correspondante jusqu'à ce que le crochet de fixation du bras inférieur glisse dans l'espace arqué à l'intérieur du crochet de fixation du bras supérieur en permettant ainsi la réalisation de la fermeture et du verrouillage du bras supérieur et du bras inférieur.

4. Clip métallique de ligature biodégradable directionnellement selon la revendication 1, ledit procédé comprenant l'utilisation de magnésium comme métal biodégradable, avec les étapes concrètes suivantes :
étape 1, extrusion de lingots de magnésium en feuilles de magnésium ;
étape 2, laminage et recuisson des feuilles de magnésium à de multiples reprises pour obtenir un matériau brut d'épaisseur requise ;
étape 3, traitement par fraiseuse CNC, découpe linéaire ou découpe au laser pour obtenir le bras supérieur, le bras inférieur et la queue de structure torique requis ;
étape 4, placement du clip de ligature précédemment préparé formé par lesdits bras supérieur, bras inférieur et queue de structure torique dans un dispositif de recuisson directionnelle pour un traitement thermique instantané afin d'établir un potentiel de dégradation différent entre la surface intérieure et extérieure du clip de manière à réaliser la fonction de dégradation directionnelle ;
ledit dispositif de recuisson directionnelle étant apte à réaliser un traitement thermique instantané pour changer directionnellement les microstructures du matériau partiel local du clip par le fait qu'à la fois la surface intérieure et la surface extérieure du bras supérieur et du bras inférieur du clip de ligature ont des microstructures différentes et qu'une granulométrie dans lesdites surfaces extérieures est supérieure à une granulométrie dans lesdites surfaces intérieures, l'épaisseur desdites microstructures différentes des deux surfaces intérieure et extérieure du clip de ligature représentant 20 % à 80 % de l'épaisseur entre la surface de contact vasculaire intérieure dudit clip de ligature et la surface de bord extérieur extrême dudit clip de ligature ;
étape 5, polissage électrique du clip de ligature préparé précédemment pour éliminer l'oxyde et les impuretés de sa surface.

5. Le procédé de préparation d'un clip métallique de ligature biodégradable directionnellement selon la revendication 4 est **caractérisé en ce que** : dans l'étape 1, ladite extrusion de métal de magnésium se produit à une température comprise entre 100° et 300 °C et avec un rapport d'extrusion de 10 à 100, et que le diamètre des grains de la feuille extrudée est ≤ 20 µm.

6. Le procédé de préparation d'un clip métallique de ligature biodégradable directionnellement selon la revendication 4 est **caractérisé en ce que** : dans l'étape 2, la déformation dudit simple laminage et de 3 % à 8 %, et la déformation cumulative avant recuisson et de 20 % à 50 % ; la recuisson centrale de la feuille se fait à une température de 130 °C à 350 °C, avec une durée de recuisson de 30 à 300 sec. ;
ladite feuille laminée ayant une épaisseur de 0,3 à 2 mm et, pour les propriétés mécaniques, une limite d'élasticité > 120 MPa, une résistance à la traction > 150 MPa et une élongation > 4 % ;
la granulométrie obtenue après ledit laminage étant de 1 à 15 µm.

7. Le procédé de préparation d'un clip métallique de ligature biodégradable directionnellement selon la revendication 4 est **caractérisé en ce que** : dans l'étape 4, lesdites microstructures différentes se réfèrent à :
(1) pour le clip de ligature en magnésium pur, les différentes microstructures de matériau se réfèrent à : 1) la granulométrie de la surface extérieure du clip de ligature est supérieure à la granulométrie de la surface intérieure ; 2) il a des densités de dislocation et un niveau de contrainte différents pour établir ainsi un potentiel de dégradation sur le clip de ligature dans sa dégradation de manière à assurer que le potentiel de dégradation sur la surface extérieure soit inférieur à ce qu'il est sur la surface intérieure ; après la fermeture du clip biodégradable, la dégradation directionnelle se déroule depuis le clip de ligature de l'extérieur vers l'intérieur ;
(2) pour le clip de ligature en alliage de magnésium, les différentes microstructures de matériau comprennent: 1) une granulométrie différente, 2) la microstructure de surface extérieure a une proportion plus élevée de seconde phase à phase précipitée que la surface intérieure, 3) une densité de dislocation et un niveau de contrainte différents pour produire ainsi la différence de potentiel de dégradation inférieure sur la surface extérieure à ce qu'elle est sur la surface intérieure.

8. Le procédé de préparation d'un clip métallique de ligature biodégradable directionnellement selon la revendication 7 est **caractérisé en ce que** : la différence de potentiel intérieur-extérieur dudit clip de ligature en magnésium pur ou en alliage de magnésium se réfère au fait que la différence de potentiel extérieure est 50 à 300 mV plus faible que l'intérieure en formant un couple de corrosion galvanique pendant la dégradation afin de dégrader ainsi préférentiellement l'extérieur et de protéger l'intérieur de manière à établir l'action de dégradation directionnelle nécessaire.

9. Le procédé de préparation d'un clip métallique de ligature biodégradable directionnellement selon l'une quelconque des revendications 4 à 7 est **caractérisé en ce que** : ladite recuisson directionnelle se réfère à ce qui suit :
mettre le clip de ligature dans le dispositif de recuisson directionnelle, et
asperger la zone intérieure du clip de ligature avec du gaz inerte au-dessus du clip de ligature aux fins de refroidissement ; en même temps, un tube d'échange thermique à advection est autour du clip de ligature à l'extérieur dans l'entrée duquel se trouve un fluide d'échange thermique à haute température à 200° à 410 °C, et transférer de la chaleur vers le clip de ligature à l'extérieur pour recuisson ; après une certaine période de recuisson, changer la direction d'une vanne variable pour le fluide d'échange thermique, et faire entrer instantanément du fluide à basse température à 5 °C à 30 °C pour le refroidissement ; finir ainsi le processus de recuisson directionnelle et de trempe.

10. Le procédé de préparation d'un clip métallique de ligature biodégradable directionnellement selon la revendication 9 est **caractérisé en ce que** : ledit dispositif de recuisson directionnelle comprend un bain de recuisson, une canalisation de fluide de recuisson posée à la périphérie du bain de recuisson de clip, le fluide de recuisson s'écoulant à la température réglée dans la canalisation de fluide de recuisson, la vanne variable à l'entrée de la canalisation de fluide de recuisson permettant un échange thermique de fluide à des températures différentes, et la canalisation de fluide étant interconnectée ;
ledit gaz inerte étant l'un ou un mélange aléatoire parmi l'azote, l'hélium, le néon, l'argon, le krypton, le xénon, le dioxyde de carbone ;
ledit fluide d'échange thermique étant l'un parmi l'huile de silicone, l'huile de trempe, l'huile de machine, une solution aqueuse à haute pression et aux températures est un gaz à haute température ;
ladite température de recuisson étant de 200 °C à 400 °C, et ladite durée de recuisson thermique étant de 10 à 300 secondes.
